(19) Europäisches Patentamt
European Patent Office
Office européen des brevets

(11) Publication number: **0 062 440**
**A1**

(12) # EUROPEAN PATENT APPLICATION

(21) Application number: 82301482.4

(22) Date of filing: 23.03.82

(51) Int. Cl.³: **C 07 C 59/52**, C 07 D 263/56, C 07 C 51/00

(30) Priority: 27.03.81 US 248379

(43) Date of publication of application: 13.10.82 Bulletin 82/41

(84) Designated Contracting States: BE CH DE FR GB IT LI LU NL SE

(71) Applicant: ELI LILLY AND COMPANY, 307, East McCarty Street, Indianapolis Indiana 46285 (US)

(72) Inventor: Greene, James Michael, 607, West Ralston Road, Indianapolis Indiana 46217 (US)

(74) Representative: Crowther, Terence Roger et al, Erl Wood Manor, Windlesham Surrey GU20 6PH (GB)

(54) **Process for preparing phenylalkanoic acids and derivatives of benzoxazole therefrom.**

(57) Hydroxyphenylacetic acids are formed from the corresponding benzaldehydes or alkyl phenyl ketones by reductive amination, cyanation and hydrolysis without isolation or purification of intermediate products. These hydroxyphenylacetic acids are useful intermediates in the preparation of pharmacologically active derivatives.

EP 0 062 440 A1

TITLE MODIFIED see front page

PROCESS FOR PREPARING PHENYLALKANOIC ACIDS

This invention belongs to the field of synthetic organic chemistry, and provides a particularly advantageous process for preparing certain phenylalkanoic acids from the corresponding benzaldehydes or phenyl ketones. The process provides the desired acids in high yield and acceptable purity without isolation of intermediate products.

This invention provides a process for preparing an acid of the formula (I)

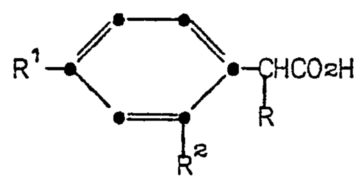

(I)

wherein R is hydrogen or $C_1$-$C_3$ alkyl; one of $R^1$ and $R^2$ is hydrogen and the other is hydroxy; provided that R is hydrogen or methyl when $R^2$ is hydroxy; comprising catalytically hydrogenating a compound of the formula

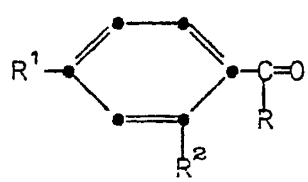

in the presence of an amine of the formula

$$HNR^3R^4$$

wherein $R^3$ is $C_1$-$C_3$ alkyl and $R^4$ is hydrogen or $C_1$-$C_3$ alkyl, in an inert organic solvent to prepare a compound of the formula

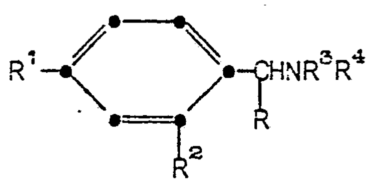

adding an alkali metal cyanide to the mixture; holding the mixture at from about 100° to about 150° until a compound of the formula

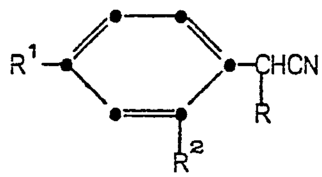

is formed;
removing the solvent;
adding aqueous alkali metal hydroxide; holding the mixture at from about 75° to about 125° until the product is formed; and
making the mixture acid.

The separation of the product acid is preferably accomplished by heating the acidified reaction mixture containing the acid of formula (I) to about 75°-125°; filtering it at about 75°-125°; cooling the mixture; extracting the mixture with an inert organic solvent; and isolating the acid from the organic solvent.

In this document, all temperatures are described in degrees Celsius. All expressions of concentrations, ratios, proportions and the like refer to measurements by weight unless otherwise stated.

In the above statement of the invention, the term $C_1$-$C_3$ alkyl refers to the groups methyl, ethyl, propyl and isopropyl.

It is believed that the compounds which are prepared by the process of this invention are entirely familiar to organic chemists. However, the following typical products will be mentioned, to assure that the invention is fully understood.

4-hydroxyphenylacetic acid
4-hydroxy-α-isopropylphenylacetic acid
4-hydroxy-α-ethylphenylacetic acid
2-hydroxy-α-methylphenylacetic acid
4-hydroxy-α-methylphenylacetic acid
4-hydroxy-α-propylphenylacetic acid
2-hydroxyphenylacetic acid

A preferred group of products of this invention includes the compounds wherein $R^1$ is hydroxy. Another preferred group of compounds includes those wherein R is hydrogen, another preferred group includes those wherein R is methyl, and a particularly preferred compound is 4-hydroxy-α-methylphenylacetic acid.

All of the starting compounds and reagents used in the process of this invention are well known to organic chemists, and can be easily purchased or prepared by methods well known in the art.

In general, only the stoichiometric amounts of the various compounds used in the process of this invention are necessary. The only important exception, as will be explained further below, is that an excess amount of base is desirable in the hydrolysis step. In

the other steps, however, the exact stoichiometric amounts may be successfully used. As is usual in organic processes, it is usually advisable to use a small excess of inexpensive reagents and starting compounds, to assure that the more expensive substances are fully consumed. For this purpose, small excesses in the range of about, for example, 1.02 to 1.25 of the stoichiometric amounts may often be economically utilized.

The process of this invention will now be explained in detail. The reader should note that the individual steps are free of isolation or purification processes, except of course for the isolation of the acid which is the product of the process. It will be observed that the process is very appropriate for use in large-scale enclosed equipment, and that none of the intermediates, reagents or solvents present any unusual hazards of flammability or toxicity. The equipment needed to carry out the process is of the types commonly found in organic chemical processing plants, and no unusual corrosion problems are presented.

The concentration of the reaction mixtures is not critical. In the various steps below, an indication of the desired concentration range is given, but it is not implied that the concentrations suggested are limitations in any way on the operability of the process.

No exact times are given for the individual steps. As is always the case in chemistry, the speeds

of the reactions depend very closely on the operating temperatures, and in part on other considerations such as the exact compound which is to be prepared. Indications of the times needed by the various steps are given below for the guidance of the reader, who will understand that the times stated are only indications of preferred conditions and that the times will vary markedly under slightly different operating conditions. An organic chemist will understand that the course of the reactions can be easily followed, as by thin layer chromatography, to tell when the reaction is as complete as he may desire. In some instances, the operator will wish to maximize the yield of the process by giving maximum periods of reaction time in each step; in other instances, he will wish to maximize throughput by cutting off each step at the point where it has reached an economical degree of completion.

In the reductive amination step, the starting compound, which is a hydroxybenzaldehyde when the group R is hydrogen, or is a phenyl alkyl ketone when R is an alkyl group, is hydrogenated catalytically in the presence of a suitable alkylamine to form the corresponding amino compound. The amine may be either a monoalkylamine, or a dialkylamine. It is preferred to use a dialkylamine when the starting compound is a hydroxybenzaldehyde, and to use a monoalkylamine when the starting compound is a ketone.

Suitable monoalkylamines include, for example, methylamine, ethylamine and propylamine; dialkylamines include ethylmethylamine, dimethylamine, diethylamine,

dipropylamine and ethylpropylamine. The preferred monoalkylamine is methylamine, and the preferred dialkylamine is dimethylamine.

The preferred solvent for the reductive amination step is dimethylformamide. It is preferred because of its high boiling point and stability, which properties make it particularly advantageous both in the amination step and in the following cyanation step. Other solvents, however, may be used in particular circumstances, particularly dimethylacetamide, hexamethylphosphoramide and dimethylsulfoxide.

The usual hydrogenation catalysts are used in the reductive amination. The preferred catalyst is carbon-supported palladium, but other catalysts, such as platinum, platinum oxide, nickel, rhodium and ruthenium may be used as desired. Hydrogen pressures from about 2 atmospheres to about 150 atmospheres are used, as is usual in the art, and the preferred process temperature is the ambient temperature. Temperatures as high even as about 100° may be used, however. A convenient concentration for the step is in the range of 1-2 gram-moles per liter, but higher and lower concentrations are readily used as may be dictated by operating convenience in a given situation.

When the reductive amination is complete, or is as complete as is desired, the reaction mixture is removed from the hydrogenator. The catalyst may be separated from it, usually by filtering or centrifuging the mixture, or may be left in the mixture to be filtered out later.

An alkali metal cyanide is added to the reaction mixture from the first step. The preferred cyanide is sodium cyanide, but other alkali metal cyanides such as potassium or lithium cyanide may be used as well if desired. The mixture is then heated to an elevated temperature in the range of from about 100° to about 150°, and is stirred at that temperature until the desired acetonitrile is formed.

The preferred temperature is in the range of from about 120° to about 150°, and the optimum reaction time, when a temperature in that range is used, is about 4 to 8 hours.

When the cyanation step has gone to the desired degree of completion, the solvent is distilled from the reaction mixture, as by putting the mixture under vacuum while the vessel is heated. It is not necessary to continue the distillation until the mixture is analytically free of solvent; the later hydrolysis step will hydrolyze and remove the remaining solvent, especially when the solvent is dimethylformamide, so that residual solvent does not interfere with the isolation of the final product.

The product from the cyanation step, after the solvent has been distilled, is a thick oily or greasy residue.

In the hydrolysis step, the phenylacetonitrile compound is converted to the desired carboxylic acid by a basic hydrolysis in the presence of an aqueous solution of an alkali metal hydroxide. The preferred base is sodium hydroxide, but potassium and

lithium hydroxides also may be used advantageously. The reaction mixture is most easily prepared by simply adding the aqueous base to the residue remaining after the distillation of solvent from the cyanation step reaction mixture.

It is advisable to use excess base in the hydrolysis. Preferably, at least about 1.5 moles of base should be used for each mole of starting compound. Larger amounts of base may be required if the residue contains residual solvent, especially dimethylformamide, which must be hydrolyzed.

The concentration of the aqueous solution of base is not critical. Relatively high concentrations, from about 10% to about 40%, are satisfactory and are preferred because of the economy and convenience of such operation. Lower concentrations can be used but are not preferred.

The basic mixture is heated to a temperature from about 75° to about 125° to accomplish the hydrolysis. Operation under pressure is of course necessary if a temperature above the boiling point is to be used. It is preferred to operate at the reflux temperature of the mixture, about 100°. Reaction times in the range of about 4 to 8 hours have been found to give essentially complete hydrolysis at the reflux temperature; operation at higher temperatures will, of course, allow shorter periods of time in the hydrolysis step.

When the hydrolysis has gone as close to completion as is desired, the reaction mixture is worked

up. The first step in the preferred workup procedure is to make the mixture acid, preferably with an inexpensive mineral acid such as hydrochloric acid. Other acids may of course be used, so long as the neutralization product of the acid and the base used in the hydrolysis step is a water-soluble salt. Accordingly, sulfuric acid, phosphoric acid and the like may be used as may be convenient in the circumstances. The mixture is preferably acidified to a pH of about 2.

In some instances, it is helpful to decolorize the product before it is isolated. Activated charcoal has been found to be effective for the purpose, and a particularly advantageous point to apply it is at the end of the hydrolysis step, while the product is still in the water solution. The decolorizing agent is conveniently added either before or after the reaction mixture is made acid, and is filtered out in the filtration step which follows.

The acid mixture is then heated to a temperature in the range of from about 75° to about 125° (operation under pressure is necessary to achieve temperatures above 100°, of course) and is held at that temperature with stirring for a brief period of time in the range of from a few minutes to an hour. The mixture is then filtered in equipment which allows it to be held at a temperature in the range of about 75-125° while it is filtered. Ordinary filter paper with a filter aid pad gives adequate separation of the insoluble impurities, with acceptable speed of filtration.

The filtrate is then cooled to a temperature which approximates ambient temperature, and is extracted with an inert organic solvent. The preferred solvents are esters, especially ethyl acetate. Other solvents can be used as well, such as ethers, including tetrahydrofuran and diethyl ether, for example. Obviously, the extracting solvent must be water-immiscible, and must have adequate solvency for the phenylacetic acid. The amount of solvent should be in the range of from about 500 ml. per gram-mole of starting compound to about 2.5 liters per gram-mole of starting compound. The exact amount of solvent is not critical, but can be adjusted as may be convenient in a given instance, and in accordance with the relative value of extremely complete extraction of the product, as compared with the cost of solvent. It is advantageous, of course, to extract the mixture 2 or more times, adding the solvent in portions.

Finally, the product phenylacetic acid is isolated from the organic solvent. The isolation may be carried out by simply evaporating the solvent, leaving the product as the residue. To do so is difficult in large scale operation, however, and any of a number of expedients may be used. For example, the solvent can be evaporated, and the product can then be taken up as a suspension in a non-solvent such as hexane and recovered by filtration or centrifugation. Alternatively, the residual product can be dissolved in a relatively weak solvent for it, such as, for example, warm toluene or xylene. The addition of an anti-solvent such as hexane or another alkane, accompanied by chilling the solution, will precipitate the product as a fine crystal, which is recovered by filtration or centrifugation. Still other expedients for isolating the product, such as evaporating the solvent from it on a heated drum dryer and scraping the product off the drum, will occur to those who are skillful in process chemistry.

As the examples below illustrate, the process of this invention will easily produce the phenylacetic acids in overall yields of 85-95%, and in excellent purity.

The products of this process can be used as intermediates for the preparation of pharmaceuticals. U.S. Reissue Patent 29,608 shows a group of benzoxazoles which are analgesics and antiinflammatories, and which have a carboxylic acid group, such as that of the 4-hydroxy acids produced by this invention, at the 5- or 6-position of their phenyl ring. The acids are converted to those benzoxazoles by the following steps:

A) Nitrating the product of this invention with mixed nitric and sulfuric acids to prepare the corresponding 4-hydroxy-3-nitrophenylacetic acid;

B) Reduction of the nitro group, as by catalytic hydrogenation or with a chemical agent such as iron-hydrochloric acid, to give the 3-amino-4-hydroxyphenylacetic acid;

C) Acylation of the amino group to give the corresponding 3-carboxamido-4-hydroxyphenylacetic acid, where the amido group bears the

group which will form the 2-substituent of the final benzoxazole;

D) Cyclization of the above compound, as at an elevated temperature or in the presence of an acidic agent to give the desired benzoxazole pharmaceutical.

Preferably, $R^3$ is a <u>para</u>-chloro substituent and $R^4$ is hydrogen, since the product of the reaction is then benoxaprofen.

Thus, in one embodiment of the invention there is provided a process for preparing benzoxazole derivatives of formula (II):

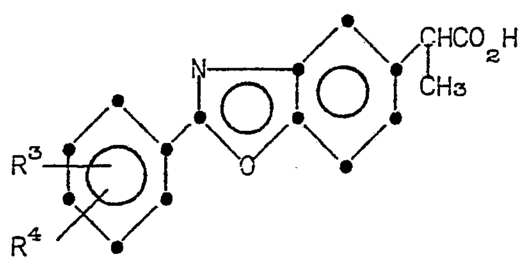

where $R^3$ and $R^4$ represent the same or different halogen atoms or hydrogen;
which comprises:

(A) nitrating an acid of formula (I), prepared as described above, in which R is methyl, $R^1$ is hydroxy and $R^2$ is hydrogen, so as to prepare the corresponding 4-hydroxy-3-nitrophenylpropionic acid;

(B) reducing the nitro group to give the corresponding 3-amino-4-hydroxyphenylpropionic acid;

(C) acylating the 3-amino group to give the corresponding 3-carboxamido-4-hydroxyphenylpropionic acid, where the amido group bears a phenyl, monohalophenyl or dihalophenyl group; and

(D) cyclizing the acylated product of step (C) to give the benzoxazole of formula (II).

The above process steps for the formation of the benzoxazole are explained, in general, in the above mentioned reissue patent.

The 4-hydroxy product of the process of this invention wherein R is hydrogen can also be used as an intermediate in preparing certain β-lactam antibiotics, particularly those taught in U.S. Patents 4,138,486 and 4,201,782, both of Shionogi and Co. The compounds of those patents are oxa-β-lactam compounds, having a carboxy(4-hydroxyphenyl)acetamido side chain. The 4-hydroxyphenylacetic acid prepared by this invention is a useful starting compound to prepare the side chain group, as explained by Greene and Bunnell in U.S. Patent Application 203,736. It is there explained that the phenylacetic acid is esterified with a benzyl halide to give the corresponding benzyl 4-hydroxyphenylacetate, the hydroxy group of which is then protected. The α-carbon of the acetate is then carboxylated, as by carbon dioxide gas in the presence of a very strong base at a very low temperature, and the

resulting carboxy(4-protected-oxyphenyl)acetate is used to acylate the nucleus of the desired antibiotic. The acylation and the necessary deprotection of the antibiotic is taught in the above-mentioned U.S. Patent 4,201,782.

The product of this process wherein R is hydrogen and $R^2$ is hydroxy is an intermediate in the synthesis of nocardicin A, a monocyclic β-lactam antibiotic, as taught by Boucherot and Pilgrim, Tet. Let. 5063-66 (1979). The compound is also useful to promote the rooting of plant cuttings, Vazquez et. al., An. Edafol. Agrobiol. 37(5-6), 441-44 (1978); C.A. 90, 17550k (1979).

The product wherein R is methyl and $R^2$ is hydroxy is an intermediate in the synthesis of certain dibenzoxepins having pharmaceutical uses. Ueno et al., German OLS 2,435,613, C.A. 82, 170741c (1975). The product is converted to an ether with 2-carboxytoluene, and the ether is cyclized with polyphosphoric acid and the resultant product is reduced.

The following non-limiting Examples will further explain the invention.

## Example 1

4-hydroxy-α-methylphenylacetic acid

A 27.2 g. portion of 4-hydroxyacetophenone was dissolved in 100 ml. of dimethylformamide, and the solution was cooled to 5°. To it were added 12.4 g. of anhydrous methylamine and 5 g. of 5% palladium/carbon hydrogenation catalyst. The mixture was put in a Parr hydrogenator under 50 psig. of hydrogen at 45°, and was

shaken at constant temperature for 18 hours. The reaction mixture was then filtered, and to the filtrate was added 10.8 g. of sodium cyanide. The mixture was heated to 140°, and was held at that temperature with stirring for 5 hours. The mixture was then put under vacuum and was distilled down to a total weight of 54 g.

To the residue was added 75 ml. of 50% aqueous sodium hydroxide and 75 ml. of water. The mixture was stirred under reflux for 8 hours. The solution was cooled to ambient temperature, and was acidified to pH 2 by the addition of concentrated hydrochloric acid. One hundred ml. of additional water was added, the mixture was heated with stirring to reflux, and 2.7 g. of activated carbon was added. The mixture was stirred at the reflux temperature for 30 minutes, and was filtered. The filtrate was cooled to near the ambient temperature, and was extracted with two 100 ml. portions of ethyl acetate. The organic layers were combined, and were evaporated under vacuum to obtain 39 g. of residue. To it was added 100 ml. of toluene, and the suspension was stirred for a time, after which 100 ml. of hexane was added. The resulting slurry was stirred in an ice bath for 1 hour and was filtered, and the solids were dried under vacuum at 45° to obtain 28.2 g. of the desired product, a yield of 85% of the theoretical yield. Its melting point was 128-130°.

Example 2

4-hydroxy-α-methylphenylacetic acid

The process of this example was carried out substantially according to the process of Example 1, except that, after the hydrolysis reaction mixture had been made acid with hydrochloric acid, and then heated to reflux, 3 g. of activated carbon was added and it was stirred under reflux for 30 minutes. The mixture was then filtered through a filter aid pad, using a steam-jacketed Buchner funnel, and the filtrate was cooled and extracted twice with 125 ml. portions of ethyl acetate. The ethyl acetate layers were combined and evaporated, and the product was recovered and dried as described in Example 1 to obtain 28.3 g. of the desired product, equivalent to 85% of the theoretical yield of the process. The product was identical to that of Example 1.

Example 3

4-hydroxyphenylacetic acid

A 24.4 g. portion of 4-hydroxybenzaldehyde was dissolved in 100 ml. of dimethylformamide, and the solution was cooled in an ice bath. To it was added 18 g. of anhydrous dimethylamine, and 0.1 g. of platinum oxide hydrogenation catalyst. The mixture was put under 50 psig of hydrogen in an agitated hydrogenator for 1 hour and 15 minutes, during which time it took up 16 psi of hydrogen, the theoretical uptake. The mixture was then filtered into another container, and the hydrogenator and filter were washed with 20 ml. of

additional dimethylformamide. To the filtrate was added 10.8 g. of sodium cyanide, and the mixture was stirred at 130° for 6 hours.

The solvent was then removed under vacuum at 80° to obtain 49.6 g. of a dark residue. To it were added 150 ml. of water and 20 g. of sodium hydroxide pellets, and the aqueous mixture was stirred under reflux for 6 hours. It was then cooled to ambient temperature, and its pH was adjusted to 1.8 by the addition of about 60 ml. of concentrated hydrochloric acid. Six g. of activated carbon was added, and the mixture was heated briefly to reflux. It was then filtered at the boiling point, and the filter was washed with 50 ml. of hot water. The filtrate was then cooled, and was extracted with three 100 ml. portions of ethyl acetate. The organic layers were combined, washed with 50 ml. of water and dried over magnesium sulfate. The organic solution was then concentrated under vacuum to a weight of 70 g., and to the residue was slowly added 150 ml. of toluene, with stirring and cooling. The mixture was then stirred for one hour and filtered, and the solids were washed with cold toluene and dried under vacuum to obtain 25.6 g. of the desired product, m.p. 149-152°. The yield was 84.4% of theoretical.

CLAIMS

1. A process for preparing an acid of the formula

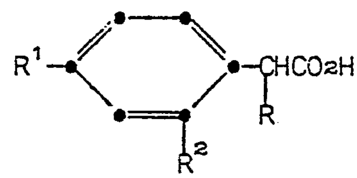

wherein R is hydrogen or $C_1$-$C_3$ alkyl; one of $R^1$ and $R^2$ is hydrogen and the other is hydroxy; provided that R is hydrogen or methyl when $R^2$ is hydroxy; comprising catalytically hydrogenating a compound of the formula

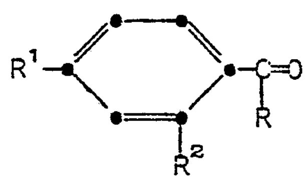

in the presence of an amine of the formula

$$HNR^3R^4$$

wherein $R^3$ is $C_1$-$C_3$ alkyl and $R^4$ is hydrogen or $C_1$-$C_3$ alkyl, in an inert organic solvent to prepare a compound of the formula

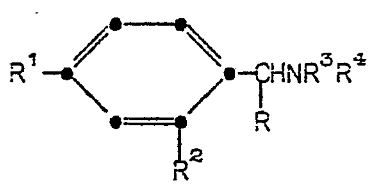

adding an alkali metal cyanide to the mixture; holding the mixture at from about 100° to about 150° until a compound of the formula

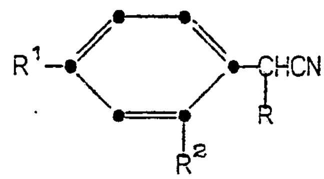

is formed;
removing the solvent;
adding aqueous alkali metal hydroxide; holding the mixture at from about 75° to about 125° until the product is formed; and
making the mixture acid.

2. A process according to claim 1, wherein R is methyl, $R^1$ is hydroxy and $R^2$ is hydrogen.

3. A process for preparing a benzoxazole of formula (II):

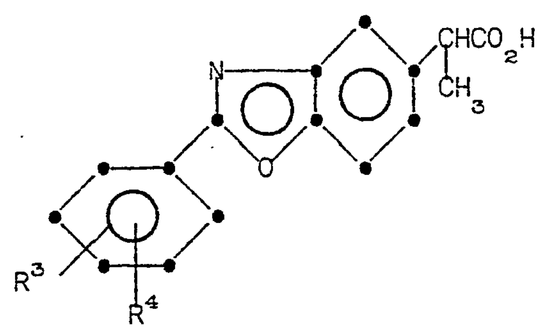

(II)

where $R^3$ and $R^4$ represent the same or different halo groups or hydrogen;
which comprises

(A) nitrating an acid of formula (I), whenever prepared by the process of claim 2, so as to prepare the corresponding 4-hydroxy-3-nitrophenylpropionic acid;

(B) reducing the nitro group to give the corresponding 3-amino-4-hydroxyphenylpropionic acid;

(C) acylating the amino group to give the corresponding 3-carboxamido-4-hydroxyphenylpropionic acid, where the amido group bears a phenyl, monohalophenyl or dihalophenyl group;

(D) cyclizing the acylated product of step (C) to give the benzoxazole of formula (II).

4. A process according to claim 3, wherein $R^3$ is para-chloro and $R^4$ is hydrogen.

European Patent Office

# EUROPEAN SEARCH REPORT

0062440

Application number

EP 82 30 1482.4

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| Y | AT - B - 336 570 (HOECHST)<br>* claim 1 *<br>-- | 1 | C 07 C 59/52<br>C 07 D 263/56<br>C 07 C 51/00 |
| Y | US - A - 4 087 437 (E.A. STEPHAN)<br>* example 1 *<br>-- | 1 | |
| A | GB - A - 1 522 477 (I.C.I.)<br>* claim 1 *<br>-- | 1 | |
| Y | DE - A1 - 2 931 255 (RAVIZZA S.P.A. PER L'INDUSTRIA CHIMICA E FARMACEUTICA)<br>* page 7, lines 25 to 30; page 8, lines 5 to 10 *<br>-- | 2,3 | TECHNICAL FIELDS SEARCHED (Int.Cl. 3)<br>C 07 C 51/00<br>C 07 C 51/08<br>C 07 C 59/52<br>C 07 C 85/08<br>C 07 C 121/75<br>C 07 D 263/56 |
| Y | GB - A - 1 435 721 (LILLY INDUSTRIES)<br>* claim 74 *<br>---- | 3 | |

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| Berlin | 24-06-1982 | KNAACK |